# EUROPEAN PATENT APPLICATION

(11) **EP 4 834 312 A2**
(43) Date of publication of application: **04.11.2026**
(21) Application number: 26175314.9
(22) Date of filing: 29.04.2026
(51) Int. Cl.: A61K 8/06, A61K 8/19, A61K 8/31, A61K 8/34, A61K 8/36, A61K 8/37, A61K 8/73, A61K 8/81, A61Q 1/00, A61Q 1/04, A61Q 1/10, A61Q 17/04, A61Q 19/00, A61Q 19/08

(54) **POLYGLYCEROL BASED COSMETIC COMPOSITION**

(30) Priority: 30.04.2025 US 202563797741 P
(71) Applicant: Shiseido Company, Limited, Chuo-Ku Tokyo 104-0061 (JP)
(72) Inventor: FURUKAWA, Ryo, East Windsor, 08520 (US)
(74) Representative: Ter Meer und Partner mbB

(57) **Abstract**

Cosmetic compositions and methods of using the cosmetic compositions are disclosed. The cosmetic compositions include compositions containing a polyglycerol surfactant, a higher alcohol, a fatty acid, and a neutralization agent.

## Description

### CROSS-REFERENCE TO RELATED APPLICATION

This application claims the benefit of priority to U.S. Provisional Application Serial No. 63/797,741, filed on April 30, 2025, which is incorporated herein by reference in its entirety.

### TECHNICAL FIELD

The present disclosure relates to polyglycerol-based cosmetic oil-in-water emulsion (O/W emulsion) compositions, and in some aspects, the polyglycerol-based composition does not contain polyethylene glycol (PEG) and/or silicone.

### BACKGROUND

The cosmetic industry is moving toward sustainability, pushed not only by industry regulations and standards (e.g. microplastics), but also by increasing concerns about the long-term disposal of the chemical substances present in cosmetic formulations and its associated adverse effects on the environment (e.g. silicones), the chemicals used during production (e.g. ethylene oxide used for PEG derivatives), the natural origin index (NOI) rating of the ingredients, the energy consumption for the production of ingredients and products, etc. Currently, there is a high demand for cosmetics with naturally derived ingredients, indicating an increasing awareness and demand from consumers concerning sustainability.

Additionally, consumers expect easy application, comfortable sensory feeling in use (feeling of skin being hydrated and soft) and composition stability. Thus, there is a need for sustainable cosmetic compositions that retain the comfortable sensory feeling in use and composition stability expected by consumers.

For these reasons, there is a need for improved natural cosmetic compositions.

### SUMMARY

An exemplary aspect of the present disclosure relates to a cosmetic, optionally dermatologically photoprotective, composition comprising: a polyglycerol surfactant, a higher alcohol, a fatty acid, and a neutralization agent. In some aspects, the cosmetic composition is essentially free of silicone and polyethylene glycol (PEG). In some aspects, the cosmetic composition is an oil-in-water (O/W) emulsion comprising particles that are less than 15 microns in size. In some aspects, the cosmetic composition further comprises a UV absorber.

In another aspect, the present disclosure is a cosmetic composition having about 0.5-5% by weight of a polyglycerol surfactant, about 1-5% by weight of a higher alcohol, about 0.05-0.5% by weight of a fatty acid, and about 0.0005-0.5% by weight of a neutralization agent.

In another aspect, the present disclosure is a cosmetic composition having about 3% by weight of a polyglycerol surfactant, about 3.6% by weight of a higher alcohol, about 0.2% by weight of a fatty acid, about 0.02% by weight of a neutralization agent, and less than 15% by weight of a UV absorber.

In another aspect, the present disclosure relates to a method of treating the skin with a cosmetic composition, wherein the method comprises topically applying to the skin of the subject the cosmetic composition of the disclosure.

Each of the aspects of the present disclosure can encompass various elements of the present disclosure. It is, therefore, anticipated that each of the aspects of the present disclosure involving any one element or combinations of elements can be included in each aspect of the present disclosure. This disclosure is not limited in its application to the details of construction and the arrangement of components set forth in the following detailed description or illustrated in the drawings.

### BRIEF DESCRIPTION OF THE DRAWINGS

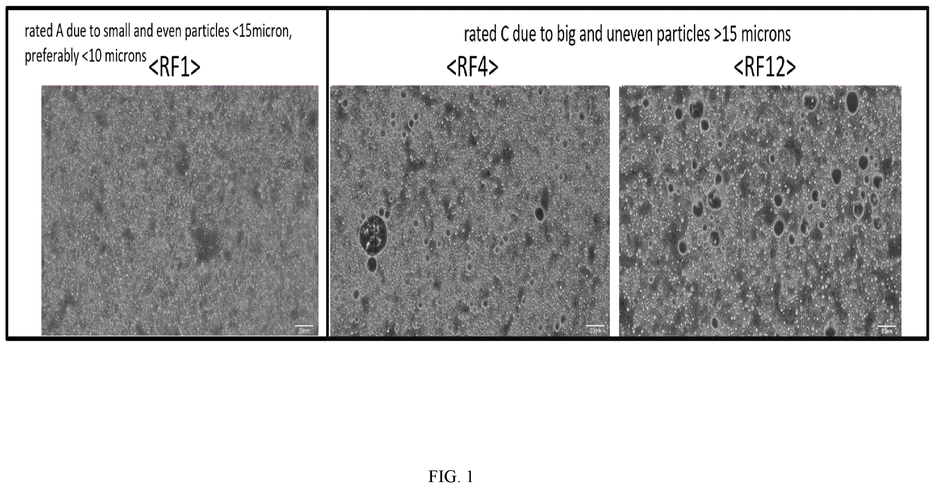

FIG. 1 shows optical microscopy images of emulsion particles of formulation RF1 compared to formulation RF4 and formulation RF12.

Note that any one or more of the illustrative components of the formulation composition are optional and the present disclosure includes aspects that contain fewer than all of the illustrated elements.

### DETAILED DESCRIPTION

The disclosure relates to a cosmetic composition comprising: a polyglycerol surfactant, a higher alcohol, a fatty acid, a neutralization agent, and an organic UV absorber, and methods of using the cosmetic composition.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 0.01 to 2.0" should be interpreted to include not only the explicitly recited values of about 0.01 to about 2.0, but also include individual values and sub-ranges within the indicated range. Thus, included in this numerical range are individual values such as 0.5, 0.7, and 1.5, and sub-ranges such as from 0.5 to 1.7, 0.7 to 1.5, and from 1.0 to 1.5, etc. Furthermore, such an interpretation should apply regardless of the breadth of the range or the characteristics being described. Additionally, it is noted that all percentages are in weight, unless specified otherwise.

In understanding the scope of the present disclosure, the terms "including" or "comprising" and their derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms "including", "having" and their derivatives. The term "consisting" and its derivatives, as used herein, are intended to be closed terms that specify the presence of the stated features, elements, components, groups, integers, and/or steps, but exclude the presence of other unstated features, elements, components, groups, integers and/or steps. The term "consisting essentially of," as used herein, is intended to specify the presence of the stated features, elements, components, groups, integers, and/or steps as well as those that do not materially affect the basic and novel characteristic(s) of features, elements, components, groups, integers, and/or steps. It is understood that reference to any one of these transition terms (i.e. "comprising," "consisting," or "consisting essentially") provides direct support for replacement to any of the other transition term not specifically used. For example, amending a term from "comprising" to "consisting essentially of" or "consisting of" would find direct support due to this definition for any elements disclosed throughout this disclosure. Based on this definition, the present disclosure supports including or excluding any element disclosed herein or incorporated by reference from the claims.

As used herein, a plurality of compounds, elements, or steps may be presented in a common list for convenience. However, these lists should be construed as though each member of the list is individually identified as a separate and unique member. Thus, no individual member of such list should be construed as a *de facto* equivalent of any other member of the same list solely based on their presentation in a common group without indications to the contrary.

Although the present disclosure is described in detail below, it is to be understood that this disclosure is not limited to the particular methodologies, protocols and/or reagents described herein as these may vary. It is also to be understood that the terminology used herein is for the purpose of describing particular aspects only, and is not intended to limit the scope of the present disclosure. Unless defined otherwise, all technical and scientific terms used herein have the same meanings as commonly understood by one of ordinary skill in the art.

While the invention is described in conjunction with the illustrated embodiments, it is understood that they are not intended to limit the invention to those embodiments. On the contrary, the invention is intended to cover all alternatives, modifications, and equivalents, including equivalents of specific features, which may be included within the invention as defined by the appended claims.

The summary and detailed description, as well as the following examples, are exemplary and explanatory only and are not restrictive of the teachings. The section headings used herein are for organizational purposes only and are not to be construed as limiting the desired subject matter in any way. In the event that any literature incorporated by reference contradicts any term defined in this specification, this specification controls. All ranges given in the application encompass the endpoints unless stated otherwise.

### Definitions

As used herein, "a" and "an" refers to one or to more than one (e.g., to at least one) of the grammatical object of the article. By way of example, "an element" means one element or more than one element.

Also as used herein, "and/or" refers to and encompasses any and all possible combinations of one or more of the associated listed items, as well as the lack of combinations when interpreted in the alternative ("or").

As used herein, the terms "about" and "approximately," when used to modify an amount specified in a numeric value or range, indicate that the numeric value as well as reasonable deviations from the value known to the skilled person in the art. For example ± 20%, ± 10%, or ± 5%, are within the intended meaning of the recited value where appropriate. [modified for clarity; please confirm that this is acceptable]

A commonly used unit of measurement for assessing the natural origin of cosmetic raw materials and of cosmetic products is the Natural Origin Index (NOI). NOI is a value indicating the extent to which a cosmetic ingredient satisfies the definition of natural ingredient, derived natural ingredient or mineral derived ingredient indicated in the ISO 16128-1 :2016 standard "Guidelines on technical definitions and criteria for natural and organic cosmetic ingredients and products - Part 1 : Definitions for ingredients" Web URL: https://www.iso.org/standard/62503.html. The NOI is a standard used in cosmetics to assess the naturalness of a product or ingredient by considering mainly the origin of the raw material and the process used for producing it. As used herein, the term "natural" refers to a material or composition with a natural origin index (NOI) of from 0.5 to 1.0, e.g., 0.5 to 1.0, 0.6 to 1.0, or 0.7 to 1.0, or 0.8 to 1.0, or 0.9 to 1.0.

As used herein a "stabilized O/W emulsion" or a "stable O/W emulsion" means a composition without an observable coalescence of the dispersed phase. In some aspects, stabilized O/W emulsion is a composition that is essentially free of phase separation. In some aspects, a stable O/W emulsion does not have phase separation observable by the naked eye. In some aspects, a stable O/W emulsion does not have microscopically observable phase separation, e.g., large and uneven particles visible using a microscope, such as a light microscope. In some aspects, a stable O/W emulsion is substantially free of particles that are more than about 15 microns in size.

As used herein the terms "polyethylene glycol" and "PEG" are intended to encompass any of the forms of polyethylene glycol (PEG). The definition thereof also includes derivatives such as , but not limited to, PEG-100 Stearate, PEG-40 hydrogenated castor oil, PEG-20 glyceryl triisostearate, PEG/PPG-17/6 copolymer.

As used herein, "UV absorber" refers to a cosmetic grade, photoprotective compound that absorbs and/or reflects some of the sun's ultraviolet (UV-A and/or UV-B) radiation and thus helps protect against negative effects of sun exposure, e.g. sunburn, premature aging, etc.

As used herein, the terms "obtained from", "derived from", "originating" and "originate" are used to identify the original source of a component but is not meant to limit the method by which the component is made which can be, for example, by chemical synthesis.

As used herein, the terms "substantially" or "essentially" refers to the qualitative condition of exhibiting total or near-total extent or degree of a characteristic or property of interest or lack of a characteristic or property of interest.

### Cosmetic Composition

In one aspect, the present disclosure relates to a cosmetic composition of the disclosure comprises a cosmetic, optionally dermatologically photoprotective, composition comprising: a polyglycerol surfactant, a higher alcohol, a fatty acid, and a neutralization agent. In some aspects, the cosmetic composition is essentially free of silicone (e.g. cylic dimethicones, linear dimethicones, silicone elastomers, silicone-based surfactants and silicone resin) and PEG. In some aspects, the cosmetic composition is an oil-in-water (O/W) emulsion. In some aspects, the emulsion is substantially free of particles that are greater than 15 microns in size. In some aspects, the emulsion is substantially free of particles that are greater than 10 microns in size.

In some aspects, the present disclosure is a cosmetic composition having about 0.1-10% by weight of a polyglycerol surfactant, about 0.1-10% by weight of a higher alcohol, about 0.01-1% by weight of a fatty acid, and about 0.0001-0.9% by weight of a neutralization agent. Preferably, the cosmetic composition has about 0.5-5% by weight of a polyglycerol surfactant, about 1-5% by weight of a higher alcohol, about 0.05-0.5% by weight of a fatty acid, and about 0.0005-0.5% by weight of a neutralization agent.

In another aspect, the present disclosure is a cosmetic composition having about 3% by weight of a polyglycerol surfactant, about 3.6% by weight of a higher alcohol, about 0.2% by weight of a fatty acid, about 0.02% by weight of a neutralization agent, and less than 15% by weight of a UV absorber.

In some aspects, the cosmetic composition comprises a thickener. For example, the cosmetic composition comprises at least one taurate based thickener and/or xanthan gum. In some aspects, the cosmetic composition comprises about 0.05-2% by weight of a taurate based thickener and/or 0.05%-0.3% by weight of xanthan gum. More preferably, the cosmetic composition comprises about 0.5% by weight of a taurate based thickener and/or 0.2% by weight of xanthan gum. In some aspects, the taurate based thickener is a copolymer of ammoniumacryloyldimethyltaurate and vinylpyrrolidone monomers (ammonium acryloyldimethyltaurate/vp copolymer). In some aspects, the cosmetic composition does not comprise a thickener.

In some aspects, the fatty acid is behenic acid. In some aspects, the neutralization agent is potassium hydroxide or sodium hydroxide. In some aspects, the higher alcohol is stearyl alcohol, behenyl alcohol or a combination thereof. In some aspects, the polyglycerol surfactant is polyglycerol-10 stearate or polyglycerol-10 di-stearate. In some aspects, the cosmetic composition further comprises a preservative. In some aspects, the preservative is phenoxyethanol.

In some aspects the percent by weight ratio of the higher alcohol to the surfactant is between 3:5 and 18:5. In some aspects, the percent by weight ratio of the neutralization agent to the fatty acid is between 1:40 and 1:1.

In some aspects, the cosmetic composition is essentially free of cyclic silicones, linear silicones, silicone resins, silicone elastomers and silicone-based surfactants. In some aspects, the cosmetic composition is essentially free of any silicones. In some aspects, the cosmetic composition is a thixotropic cosmetic composition. In some aspects, the cosmetic composition is opaque.

In some aspects, the cosmetic composition is chemically stable for at least 36 months at room temperature. In some aspects, the cosmetic composition maintains an O/W emulsion that is substantially free of particles that are between 100-25 microns, preferably between 100-20, more preferably between 100-15 in size at room temperature. In some aspects, the cosmetic composition maintains an O/W emulsion that is substantially free of particles that are greater than approximately 25 microns in size at room temperature. In some aspects, the cosmetic composition maintains an O/W emulsion that is substantially free of particles that are greater than approximately 20 microns in size at room temperature. Preferably, the cosmetic composition maintains a stabilized O/W emulsion, wherein the emulsion is substantially free of particles that are greater than 15 microns at room temperature, more preferably, the emulsion maintains an average particle size of 10 microns. Most preferably, the cosmetic composition maintains a stabilized O/W emulsion, wherein the emulsion is substantially free of particles that are greater than approximately 10 microns at room temperature.

In some aspects, the cosmetic composition of the disclosure further contains one or more coloring materials and/or dermatologically active ingredients.

The cosmetic composition may further contain any desired ingredients used in conventional cosmetic compositions. Such ingredients may include fat and oil ingredients, organic and inorganic pigments, organic powder, preservatives, antioxidants, plant extract, pH modifiers, alcohols, fragrances, antiaging ingredients, emollients, humectants, hydrating active ingredients, coolants, antiperspirants, purified water, extracts optionally within a solvent such as water or oil (e.g., ginseng water) or the like. Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

Also provided herein is a cosmetic product comprising the cosmetic composition according to any one or combination of the aspects disclosed herein. In one aspect, the cosmetic product comprising the cosmetic composition of the disclosure is a makeup cosmetic such as a foundation, such as, a liquid foundation, a BB ("blemish balm") cream or a CC ("color control") cream. In some aspects, the cosmetic product comprising the cosmetic composition of the disclosure is a skin care base, suncare, sunscreen, lipstick, lip gloss, eyeshadow, concealer, blush, highlighter, a skincare base, a skin care primer, a makeup base, or a makeup primer. In some aspects, the cosmetic composition has a NOI of from 0.5 to 1.0.

### Fatty Acid

In one aspect, the cosmetic composition of the disclosure comprises a fatty acid. The fatty acid is of a type that can be used in cosmetics or the like, and may be selected from among linear or branched fatty acids having saturated or unsaturated hydrocarbon groups.

Particular examples of fatty acids include linear higher fatty acids that are solid at room temperature and that have 8 to 22 carbon atoms such as, for example, lauric acid, myristic acid, palmitic acid, stearic acid, behenic acid and oleic acid. In some embodiments, the fatty acid may include at least one fatty acid selected from the group consisting of stearic acid, palmitic acid and behenic acid.

In some aspects, the fatty acid is a saturated fatty acid or unsaturated fatty acid. In some aspects, the fatty acid may include at least one fatty acid having from about 14 to 22 carbon atoms or from 16 to 22 carbon atoms or from 14 to 20 carbon atoms. In some aspects, the fatty acid may include at least one saturated fatty acid having from about 14 to 22 carbon atoms or from 16 to 22 carbon atoms or from 14 to 20 carbon atoms. In some aspects, the fatty acid may include at least one linear and saturated fatty acid having from about 14 to 22 carbon atoms or from 16 to 22 carbon atoms or from 14 to 20 carbon atoms. In some aspects, the fatty acid may be selected from lauric acid, tridecylic acid, myristic acid, pentadecylic acid, palmitic acid, margaric acid, stearic acid, nonadecylic acid, arachidic acid, heneicosylic acid, behenic acid, tricosylic acid, lignoceric acid and their combinations. In some aspects, the fatty acid may comprise behenic acid.

The content of the fatty acid in the cosmetic composition may vary according to the desired application. In some aspects, an amount of the fatty acid may be from 0.01-0.9% by weight; from 0.05% to 0.4% by weight; from 0.05% to 0.3% by weight; from 0.1% to 0.2% by weight; from 0.1% to 0.3% by weight; from 0.05% to 0.5% by weight; from 0.1% to 0.4% by weight; from 0.2% to 0.5% by weight; or preferably from 0.05% to 0.5% by weight or any value or subrange within these ranges.

### Neutralization Agent

In one aspect, the cosmetic composition of the disclosure comprises a neutralization agent. The neutralization agent may be an inorganic water-soluble base, such as ammonium hydroxide, potassium hydroxide or sodium hydroxide. In some aspects, the neutralization agent may include at least one of potassium hydroxide and sodium hydroxide.

The content of the neutralization agent in the cosmetic composition may vary. In some aspects, an amount of the neutralization agent may be from 0.0001% to 0.8% by weight; from 0.0005% to 0.4% by weight; from 0.005% to 0.3% by weight; from 0.01% to 0.2% by weight; from 0.1% to 0.3% by weight; from 0.005% to 0.5% by weight; from 0.01% to 0.4% by weight; from 0.02% to 0.5% by weight; or preferably from 0.0005% to 0.5% by weight or any value or subrange within these ranges.

In some aspects, the neutralization agent may be originally in the oil phase or water phase of the composition. However, the fatty acid neutralized by the neutralizing agent may be interphase, i.e. between the oil phase and the water phase.

### UV Absorber

In some aspects, the cosmetic composition further comprises an UV absorber. In some aspects, the cosmetic composition comprises less than 25% by weight of the UV absorber. In some aspects, the cosmetic composition comprises less than20% by weight of the UV absorber. In some aspects, the UV absorber absorbs ultraviolet light between about 290-400 nanometers.

It is well-known that ultraviolet radiation from sunlight also has a damaging effect on the skin. Several topical light protection filter substances have been developed to protect the skin from UVB and UVA. In general, the light absorption behavior of UV absorbers are well known and documented, in fact, most industrialized nations have lists of such substances and apply strict standards for their documentation.

In some aspects, the UV absorber may include one or more UV scattering agents. Non-limiting examples of include fine particles of metal oxides such as zinc oxide, titanium oxide, iron oxide, cerium oxide, titanium dioxide and tungsten oxide. It is furthermore known that the addition of an inorganic pigment, in particular of a titanium dioxide (TiO₂) pigment, enables the light-protection properties of sunscreens comprising UV absorbers to be improved.

In some aspects, the UV absorber may include one or more water soluble ultraviolet absorbers, which may be dissolved in the water phase of the composition. A non-limiting example of water-soluble ultraviolet absorbers is henylbenzimidazole sulfonic acid.

In some aspects, the UV absorber may include one or more oil-soluble ultraviolet absorbers, which may be dissolved in the oil phase of the composition. Examples of oil-soluble UV absorber include, but not limited to, benzoic acid, benzoic acid derivatives, salicylic acid derivatives, cinnamic acid derivatives, dibenzoylmethane derivatives, P-P-diphenyl acrylate derivatives, benzophenone derivatives, benzylidene camphor derivatives, phenylbenzimidazole derivatives, triazine derivatives, phenylbenzotri azole derivatives, anthranil derivatives, imidazoline derivatives, benzal malonate derivatives, and 4,4-diarylbutadiene derivatives.

In some aspects, the oil soluble UV absorber is homosalate, octocrylene, ethylhexyl salicylate, butylmethoxy dibenzoylmethane, bis-ethylhexyloxyphenol methoxyphenyl triazine or a combination thereof.

The content of the UV absorber in the cosmetic composition may vary depending on the application. In some aspects, an amount of the UV absorber may be 0%. In some aspects, an amount of the UV absorber may be less than 1% by weight; less than 2% by weight; less than 3% by weight; less than 4% by weight; less than 5% by weight; less than 6% by weight; less than 8% by weight; less than 10% by weight; less than 12% by weight; less than 15% by weight; less than 20% by weight; less than 25% by weight; less than 30% by weight; preferably less than 25% or more preferably less than 15% by weight; or any value or subrange of weight percentage within these ranges.

Thus, it is possible to influence the degree of shielding of the UV light through appropriate selection of the UV absorber and its concentration in the sunscreen composition. To achieve high UV protection, emulsions comprising uniformly distributed emulsion particles are commonly used.

### Emulsion

In one aspect, the cosmetic composition of the disclosure is an emulsion. In one aspect, the cosmetic composition of the disclosure comprises a polyglycerol surfactant.

In one aspect, emulsions comprising uniformly distributed emulsion particles improve stability. In an emulsion, one of the two liquids (water "W" or oil "O") is dispersed in the form of very fine droplets in the other liquid. In particular, W/O, O/W, O/W/O or W/O/W emulsions are commonly used in cosmetic compositions. Unstable composition show coalescence of the dispersed phase into larger and larger particles, which manifests into phase separation. This process can initially be observed microscopically as unstable compositions often show large and uneven particles - a sign of coalescence. Eventually, the coalescence will reach a level of phase separation that can be observed by the naked eye. Therefore, emulsions comprising uniformly sized small particles are more stable compared to an emulsion comprising larger particles.

The cosmetic composition of the disclosure advantageously achieves an even distribution of emulsion particles in an oil-in-water (O/W) emulsion, wherein the O/W emulsion is substantially free of particles that are more than about 20 microns in size. More preferably, the O/W emulsion is substantially free of particles that are more than about 15 microns in size. Most preferably, the O/W emulsion is substantially free of particles that are more than about 10 microns in size. In some aspects, the average particle size is approximately 15 microns; preferably, the average particle size is approximately 10 microns; most preferably, the average particle size is below 10 microns.

Customary emulsifiers in cosmetics often comprise hydrophilic PEG groups, which can be produced by polymerization of ethylene oxide obtained by petrochemical means. Emulsifiers obtained from renewable sources known in the art include glyceryl stearate citrate (Imwitor^{®}) and polyglycerol surfactant, such as polyglyceryl-10 stearate. Polyglyceryl-10 stearate is a hydrophilic emulsifier. However, a challenge of using the polyglycerol surfactants described in the prior art as an emulsifier in a cosmetic formulation is that it is difficult to produce stable emulsions with a desired particle size with a good formula texture and skin feel, particularly in conjunction with high microbiological stability. The cosmetic composition of the disclosure advantageously achieves an even distribution and small size of emulsion particles with no large droplets, and a rich, watery texture. In some aspects, the polyglycerol surfactant and fatty acid function as emulsifiers in the cosmetic composition of the disclosure. In some aspects, the emulsifiers are essentially free of any PEG and PEG derivatives, including PEG-100 Stearate, PEG-40 hydrogenated castor oil, PEG-20 glyceryl triisostearate, PEG/PPG-17/6 copolymer. In some aspects, the emulsifiers are essentially free of PEG.

The cosmetic composition of the disclosure further advantageously have excellent storage stability, is PEG-free, have a good skin feel, and can stabilize the emulsion against emulsion-burdening ingredients such as UV absorbers, dermatological active ingredients and pigment-containing ingredients.

### Oil

The oil phase of the emulsion comprises at least one oil. In some aspects, the oil is a hydrocarbon oil.

The term "hydrocarbon oil" may mean an oil formed essentially from, indeed even composed of, carbon and hydrogen atoms and optionally oxygen atoms and/or hydroxy groups and/or carboxyl groups. In some aspects, the hydrocarbon oil contains C13-C35 carbon atoms.

In some aspects, the oil may comprise squalane and at least one alcohol ester. In some aspects, the at least one oil may comprise at least one hydrocarbon. In some aspects, the oil is a higher alcohol. In some aspects, the higher alcohol is stearyl alcohol, behenyl alcohol or a combination thereof.

Other non-limiting examples of oils useful in the invention include aliphatic hydrocarbons such as liquid paraffin and squalane; vegetable oils, such as olive oil, almond oil, sesame oil, avocado oil, castor oil, cacao butter and palm oil; waxes including not animal derived waxes, for example carnauba wax, jojoba wax and candelilla wax; fatty acids such as lauric acid, myristic acid, palmitic acid, stearic acid, oleic acid and behenic acid; higher alcohols such as lauryl alcohol, stearyl alcohol, cetyl alcohol, behenyl alcohol and oleyl alcohol; and aliphatic esters such as isopropyl-, isocetyl- or octadecyl myristate, butyl stearate, hexyl laurate, diisopropyl adipate or diisopropyl sebacate.

### Use of the Cosmetic Composition

Also provided herein is a method of treatment and/or care of the skin, hair, nails and/or mucous membranes of a subject comprising applying the cosmetic composition of any one or combination of aspects disclosed herein to the skin, hair, nails and/or mucous membranes of the subject. In some aspects, the method comprises applying a cosmetic composition of the disclosure to a human skin.

In one aspect, the present disclosure provides a method of treating skin with a cosmetic composition, comprising applying the cosmetic composition of the disclosure comprising a low molecular weight polyether, a solvent, a thickener and a dermatological active ingredient.

While the inventions are described in conjunction with the illustrated embodiments, it is understood that they are not intended to limit the invention to those embodiments. On the contrary, the disclosure is intended to cover all alternatives, modifications, and equivalents, including equivalents of specific features, which may be included within the inventions as defined by the appended claims.
Both the foregoing general description and detailed description, as well as the following examples, are exemplary and explanatory only and are not restrictive of the teachings. The section headings used herein are for organizational purposes only and are not to be construed as limiting the desired subject matter in any way. In the event that any literature incorporated by reference contradicts any term defined in this specification, this specification controls. All ranges given in the application encompass the endpoints unless stated otherwise.

### EXAMPLES

The present invention will now be described in further detail with reference to Examples. The amount of each component is indicated in % by mass based on the total amount of the composition in which the component is blended, unless otherwise specified. The foregoing Examples are intended to illustrate and not limit the scope of the present disclosure. It will be apparent to those skilled in the art that many modifications, both to materials and methods, may be practiced without departing from the scope of the present disclosure.

### Example 1: Method of Preparation

A cosmetic composition of the disclosure having the formula shown in Table 1 was prepared by adding phase A at room temperature into a Labolution (PRIMIX Corporation) homogenizer and mixed until uniform at 75°C at the speed 4000 rpm. Phase B was added into the homogenizer and mixed for 1 min/kg at 8000 rpm until completely homogeneous at a temperature of 75 °C. Finally, the batch was cooled to 35 °C with cold water by stirring by hand.

The resulting solution (hereinafter "RF1") shows a homogenized, stable composition with small and even particles. As shown in FIG. 1, the average particle size of the RF1 composition is below 10 µm, and there are no emulsion particles larger than 15 µm. An abundance of particles bigger than 15µm in a cosmetic composition of the disclosure are considered to be unstable.

As shown in Table 1 below, RF1 was rated on a scale of A to C based on observation under optical microscopy and assessment for texture by rubbing the composition on skin. The tested composition is rated A if the composition shows small and even distribution of particles under optical microscopy and if the composition provides a rich and watery texture on the skin. The tested composition is rated C if the composition shows irregular particle size and the composition does not provide a rich and watery texture on the skin. The composition is rated B if it is intermediate of A and C.

**Table 1**

| Ingredient Type (Phase) | Trade name | INCI name | Manufacturer | RF1 |
|---|---|---|---|---|
| Water (A) | DEIONIZED WATER | WATER | N/A | 61.5 6 |
| Humectant (A) | GLYCERINE 99.7%/MB | GLYCERINE | IOI ACIDCHEM SDN BHD | 5 |
| | 1,3 BUTYLENE GLYCOL | BUTYLENE GLYCOL | OXEA CORPORATION | 5 |
| Thickener (A) | ARISTOFLEX AVC | AMMONIUM ACRYLOYLDIMETHYL TAURATE/VP COPOLYMER | CLARIANT CORPORATION | 0.5 |
| | CARBOPOL 980 POLYMER | CARBOMER | LUBRIZOL ADVANCED MATERIALS | |
| | K-OB | XANTHAN GUM | CP KELCO U.S.,INC | |
| Surfactant (B) | NIKKOL DECAGLYN 1-SV | POLYGLYCERYL-10 STEARATE | NIKKO CHEMICALS CO.,LTD | 3 |
| Neutralizer (A) | POTASSIUM HYDROXIDE | POTASSIUM HYDROXIDE | EKA CHEMICALS AB. | 0.02 |
| Oil (B) | LANETTE^{®} 18 | STEARYL ALCOHOL | BASF CORPORATION | 2.4 |
| | NIKKOL BEHENYL ALCOHOL | BEHENYL ALCOHOL | NIPPON SURFACTANT INDUSTRIES | 1.2 |
| | REFINED CETANOL | CETYL ALCOHOL | NEW JAPAN CHEMICAL CO. | |
| | NAA-222S BEADS (JSQI) | BEHENIC ACID | NOF CORPORATION | 0.2 |
| | NEOSSANCE^{™} SQUALANE | SQUALANE | APRINNOVA, LLC. | 7 |
| UV Absorber (B) | NEO HELIOPAN^{®} HMS 182573 | HOMOSALATE | SYMRISE INCORPORATED | 4 |
| | PARSOL^{®} 340 | OCTOCRYLENE | DSM NUTRITIONAL PRODUCTS LTD. | 2 |
| | NEO HELIOPAN^{®} OS | ETHYLHEXYL SALICYLATE | SYMRISE INCORPORATED | 5 |
| | PARSOL^{®} 1789 | AVOBENZONE | DSM NUTRITIONAL PRODUCTS LTD. | 2 |
| (A) | CITRIC ACID ANHYDROUS (USP) | CITRIC ACID | ARCHER DANIELS MIDLAND COMPANY | 0.02 |
| | SODIUM CITRATE | SODIUM CITRATE | ARCHER DANIELS MIDLAND COMPANY | 0.08 |
| | SODIUM HEXAMETAPHOSPHATE | SODIUM METAPHOSPHATE | KOKUSAN CHEMICAL CO., LTD | 0.02 |
| Preservative (A) | PHENOXETOL NF 272556 | PHENOXYETHANOL | THOR SPECIALTIES | 1 |
| | | | | 100 |
| Particle size (after cooling) | | | | A |
| Texture (Rich and Watery sensation) | | | | A |
| ratio for Higher Alcohol/ Surfactant | | | | 1.2 |
| ratio for KOH/ fatty acid | | | | 0.1 |

As shown in FIG. 1, RF1 was observed and imaged under optical microscopy immediately after preparation and cooling to 35 °C. As shown in FIG. 1 and Table 1, RF1 is rated A.

### Example 2: Effects of Formulation Components

As shown in Table 2, RF1 is the composition described in Example 1; RF4 shows the effect of behenic acid; and RF5, RF15 and RF16 show the effect of the base/neutralizing agent. The formulation of each sample and the effect of removing the behenic acid (RF4) and the base/neutralizing agent (RF5, RF15 and RF16) is shown in Table 2 below. Particle size and texture ratings are defined as in Example 1.

**Table 2**

| Ingredient Type/Phase | Trade name | INCI name | RF1 | RF4 | RF5 | RF1 5 | RF1 6 |
|---|---|---|---|---|---|---|---|
| Water (A) | DEIONIZED WATER | WATER | 61.5 6 | 61.7 6 | 61.5 8 | 61.3 8 | 61.5 75 |
| Humectant (A) | GLYCERINE 99.7%/MB | GLYCERINE | 5 | 5 | 5 | 5 | 5 |
| | 1,3 BUTYLENE GLYCOL | BUTYLENE GLYCOL | 5 | 5 | 5 | 5 | 5 |
| Thickener (A) | ARISTOFLEX AVC | AMMONIUM ACRYLOYLDIME THYLTAURATE/ VP COPOLYMER | 0.5 | 0.5 | 0.5 | 0.5 | 0.5 |
| | CARBOPOL 980 POLYMER | CARBOMER | | | | | |
| | K-OB | XANTHAN GUM | | | | | |
| Surfactant (B) | NIKKOL DECAGLYN 1-SV | POLYGLYCERYL -10 STEARATE | 3 | 3 | 3 | 3 | 3 |
| Neutralizer (A) | POTASSIUM HYDROXIDE | POTASSIUM HYDROXIDE | 0.02 | 0.02 | | 0.2 | 0.00 5 |
| Oil (B) | LANETTE^{®} 18 | STEARYL ALCOHOL | 2.4 | 2.4 | 2.4 | 2.4 | 2.4 |
| | NIKKOL BEHENYL ALCOHOL | BEHENYL ALCOHOL | 1.2 | 1.2 | 1.2 | 1.2 | 1.2 |
| | REFINED CETANOL | CETYL ALCOHOL | | | | | |
| | NAA-222S BEADS (JSQI) | BEHENIC ACID | 0.2 | | 0.2 | 0.2 | 0.2 |
| | NEOSSANCE^{™} SQUALANE | SQUALANE | 7 | 7 | 7 | 7 | 7 |
| UV Absorber (B) | NEO HELIOPAN^{®} HMS 182573 | HOMOSALATE | 4 | 4 | 4 | 4 | 4 |
| | PARSOL^{®} 340 | OCTOCRYLENE | 2 | 2 | 2 | 2 | 2 |
| | NEO HELIOPAN^{®} OS | ETHYLHEXYL SALICYLATE | 5 | 5 | 5 | 5 | 5 |
| | PARSOL^{®} 1789 | AVOBENZONE | 2 | 2 | 2 | 2 | 2 |
| (A) | CITRIC ACID ANHYDROUS (USP) | CITRIC ACID | 0.02 | 0.02 | 0.02 | 0.02 | 0.02 |
| | SODIUM CITRATE | SODIUM CITRATE | 0.08 | 0.08 | 0.08 | 0.08 | 0.08 |
| | SODIUM HEXAMETAPHOSPHA TE | SODIUM METAPHOSPHAT E | 0.02 | 0.02 | 0.02 | 0.02 | 0.02 |
| Preservative (A) | PHENOXETOL NF 272556 | PHENOXYETHAN OL | 1 | 1 | 1 | 1 | 1 |
| | | | 100 | 100 | 100 | 100 | 100 |
| Particle size (after cooling) | | | A | C | C | A | A |
| Texture (rich and watery sensation) | | | A | A | A | A | A |
| Ratio for Higher Alcohol/ Surfactant | | | 1.2 | 1.2 | 1.2 | 1.2 | 1.2 |
| Ratio for KOH/ fatty acid | | | 0.1 | N/A | N/A | 1 | 0.02 5 |

R4 shows that, when an oil component, specifically behenic acid, is removed, the particle size was irregular in the composition (particle size rating "C"). R5 show that when the neutralization agent, specifically potassium hydroxide, is removed, the stability of the composition is reduced (particle size rating "C"). RF15 and RF16 show that the formulation shows small and even distribution of particles and provides a rich and watery texture on the skin when the ratio of the neutralizing agent and fatty acid is between 0.025-1.

**Table 3**

| Ingredient Type/Phase | Trade name | INCI name | RF 1 | RF 2 | RF 10 | RF 3 | RF 6 | RF 7 |
|---|---|---|---|---|---|---|---|---|
| Water (A) | DEIONIZED WATER | WATER | 61. 56 | 63. 06 | 63. 56 | 64. 56 | 63. 36 | 65. 16 |
| Humectant (A) | GLYCERINE 99.7%/MB | GLYCERINE | 5 | 5 | 5 | 5 | 5 | 5 |
| | 1,3 BUTYLENE GLYCOL | BUTYLENE GLYCOL | 5 | 5 | 5 | 5 | 5 | 5 |
| Thickener (A) | ARISTOFLEX AVC | AMMONIUM ACRYLOYLDIMETHY LTAURATE/VP COPOLYMER | 0.5 | 0.5 | 0.5 | 0.5 | 0.5 | 0.5 |
| | CARBOPOL 980 POLYMER | CARBOMER | | | | | | |
| | K-OB | XANTHAN GUM | | | | | | |
| Surfactant (B) | NIKKOL DECAGLYN 1-SV | POLYGLYCERYL-10 STEARATE | 3 | 1.5 | 1 | | 3 | 3 |
| Neutralizer (A) | POTASSIUM HYDROXIDE | POTASSIUM HYDROXIDE | 0.0 2 | 0.0 2 | 0.0 2 | 0.0 2 | 0.0 2 | 0.0 2 |
| Oil (B) | LANETTE^{®} 18 | STEARYL ALCOHOL | 2.4 | 2.4 | 2.4 | 2.4 | 1.2 | |
| | NIKKOL BEHENYL ALCOHOL | BEHENYL ALCOHOL | 1.2 | 1.2 | 1.2 | 1.2 | 0.6 | |
| | REFINED CETANOL | CETYL ALCOHOL | | | | | | |
| | NAA-222S BEADS (JSQI) | BEHENIC ACID | 0.2 | 0.2 | 0.2 | 0.2 | 0.2 | 0.2 |
| | NEOSSANCE^{™} SQUALANE | SQUALANE | 7 | 7 | 7 | 7 | 7 | 7 |
| UV Absorber (B) | NEO HELIOPAN^{®} HMS 182573 | HOMOSALATE | 4 | 4 | 4 | 4 | 4 | 4 |
| | PARSOL^{®} 340 | OCTOCRYLENE | 2 | 2 | 2 | 2 | 2 | 2 |
| | NEO HELIOPAN^{®} OS | ETHYLHEXYL SALICYLATE | 5 | 5 | 5 | 5 | 5 | 5 |
| | PARSOL^{®} 1789 | AVOBENZONE | 2 | 2 | 2 | 2 | 2 | 2 |
| (A) | CITRIC ACID ANHYDROUS (USP) | CITRIC ACID | 0.0 2 | 0.0 2 | 0.0 2 | 0.0 2 | 0.0 2 | 0.0 2 |
| | SODIUM CITRATE | SODIUM CITRATE | 0.0 8 | 0.0 8 | 0.0 8 | 0.0 8 | 0.0 8 | 0.0 8 |
| | SODIUM HEXAMETAPHOSP HATE | SODIUM METAPHOSPHATE | 0.0 2 | 0.0 2 | 0.0 2 | 0.0 2 | 0.0 2 | 0.0 2 |
| Preservative (A) | PHENOXETOL NF 272556 | PHENOXYETHANOL | 1 | 1 | 1 | 1 | 1 | 1 |
| | | | 10 0 | 10 0 | 10 0 | 10 0 | 10 0 | 10 0 |
| Particle size (after cooling) | | | A | A | A | C | A | C |
| Texture (rich and watery sensation) | | | A | A | A | A | C | C |
| Ratio for Higher Alcohol/ Surfactant | | | 1.2 | 1.2 | 1.2 | 2.4 | 3.6 | N/ A |
| Ratio for KOH/ fatty acid | | | 0.1 | N/ A | N/ A | 0.1 | 0.1 | 0.1 |

RF2, RF3 and RF10 show that, when the surfactant, specifically polyglyceryl-10 stearate, is removed, the particles are irregular, and the stability of the composition and quality of the emulsion are both reduced. RF7 shows that when the higher alcohol components are removed, the particles are irregular, and the stability of the composition is reduced (both texture and particle size rating are "C"). RF6 shows that when the higher alcohols components are reduced below 2%, the stability of the composition is maintained, but the sensory texture rating of the formulation is reduced (texture rating "C").

While the subject matter of this disclosure has been described and shown in considerable detail with reference to certain illustrative aspects, including various combinations and sub-combinations of features, those skilled in the art will readily appreciate other aspects and variations and modifications thereof as encompassed within the scope of the present disclosure. Moreover, the descriptions of such aspects, combinations, and sub-combinations is not intended to convey that the claimed subject matter requires features or combinations of features other than those expressly recited in the claims. Accordingly, the scope of this disclosure is intended to include all modifications and variations encompassed within the spirit and scope of the following appended claims. Section headings, the materials, methods, and examples are illustrative only and not intended to be limiting. Other aspects, advantages, and modifications are within the scope of the following claims.
The invention is illustrated by the following items:
1. A cosmetic composition comprising:
   a. a polyglycerol surfactant,
   b. a higher alcohol,
   c. a fatty acid, and
   d. a neutralization agent,
   wherein the cosmetic composition is an oil-in-water (O/W) emulsion, wherein the emulsion is substantially free of particles that are greater than 15 microns in size.
2. A cosmetic composition comprising:
   a. about 0.5-5% by weight of a polyglycerol surfactant,
   b. about 1-5% by weight of a higher alcohol,
   c. about 0.05-0.5% by weight of a fatty acid, and
   d. about 0.0005-0.5% by weight of a neutralization agent,
   wherein the cosmetic composition is an oil-in-water (O/W) emulsion, wherein the emulsion is substantially free of particles that are greater than 15 microns in size.
3. The cosmetic composition of item 1 or 2, comprising:
   a. about 1-3% by weight of a polyglycerol surfactant,
   b. about 2-3.8% by weight of a higher alcohol,
   c. about 0.1-0.3% by weight of a fatty acid, and
   d. about 0.05-0.2% by weight of a neutralization agent.
4. The cosmetic composition of any one of items 1-3, wherein the cosmetic composition further comprises an UV absorber.
5. The cosmetic composition of any one of items 1-3, wherein the cosmetic composition comprises less than 25% by weight of a UV absorber.
6. The cosmetic composition of item 5, wherein the cosmetic composition comprises less than 20% by weight of the UV absorber.
7. The cosmetic composition of any one of items 1-6, wherein the cosmetic composition does not comprise a thickener.
8. The cosmetic composition of any one of items 1-6, wherein the cosmetic composition further comprises at least one taurate based thickener and/or xanthan gum.
9. The cosmetic composition of any one of items 1-6, wherein the cosmetic composition comprises about 0.05-2% by weight of a taurate based thickener and/or 0.05%-0.3% by weight of xanthan gum.
10. The cosmetic composition of item 9, wherein the cosmetic composition comprises about 0.5% by weight of a taurate based thickener and/or 0.2% by weight of xanthan gum.
11. The cosmetic composition of any one of items 8-10, wherein the taurate based thickener is a copolymer of ammoniumacryloyldimethyltaurate and vinylpyrrolidone monomers (ammonium acryloyldimethyltaurate/vp copolymer).
12. The cosmetic composition of any one of items 1-11, wherein the emulsion is substantially free of particles that are greater than 10 microns in size.
13. The cosmetic composition of any one of items 1-12, wherein the fatty acid is behenic acid.
14. The cosmetic composition of any one of items 1-13, wherein the neutralization agent is potassium hydroxide or sodium hydroxide.
15. The cosmetic composition of any one of items 1-14, wherein the higher alcohol is stearyl alcohol, behenyl alcohol or a combination thereof.
16. The cosmetic composition of any one of items 1-15, wherein the polyglycerol surfactant is polyglycerol-10 stearate or polyglycerol-10 di-stearate.
17. The cosmetic composition of any one of items 4-16, wherein the UV absorber is homosalate, octocrylene, ethylhexyl salicylate, butylmethoxy dibenzoylmethane, bis-ethylhexyloxyphenol methoxyphenyl triazine or a combination thereof.
18. The cosmetic composition of any one of items 4-17, wherein the UV absorber absorbs ultraviolet light between about 290-400 nanometers.
19. The cosmetic composition of any one of items 1-18, wherein the cosmetic composition further comprises a preservative.
20. The cosmetic composition of item 19, wherein the preservative is phenoxyethanol.
21. The cosmetic composition of any one of items 1-20, wherein the percent by weight ratio of the higher alcohol to the polyglycerol surfactant is between 3:5 and 18:5.
22. The cosmetic composition of any one of items 1-21, wherein the percent by weight ratio of the neutralization agent to the fatty acid is between 1:40 and 1:1.
23. The cosmetic composition of any one of items 1-22, wherein the cosmetic composition is essentially free of PEG
24. The cosmetic composition of any one of items 1-23, wherein the cosmetic composition is essentially free of any silicones.
25. The cosmetic composition of any one of items 1-23, wherein the cosmetic composition is essentially free of cyclic silicones, linear silicones, silicone resins, silicone elastomers, and silicone-based surfactants.
26. The cosmetic composition of any one of items 1-25, wherein the cosmetic composition is a thixotropic cosmetic composition.
27. The cosmetic composition of any one of items 1-26, wherein the cosmetic composition is opaque.
28. The cosmetic composition of any one of items 1-27, wherein the cosmetic composition is chemically stable for at least 36 months at room temperature.
29. The cosmetic composition of any one of items 1-28, wherein the cosmetic composition maintains a stabilized O/W emulsion.
30. The cosmetic composition of any one of items 1-29, further containing one or more coloring materials and/or dermatological active ingredients.
31. The cosmetic composition of item 30, wherein the dermatological active ingredients are emollients, humectants, hydrating active ingredients, antiaging ingredients, extracts or combinations thereof.
32. A cosmetic product comprising the cosmetic composition of any one of items 30-31, wherein the cosmetic product is selected from a skin care base, suncare, sunscreen, lipstick, a foundation, lip gloss, eyeshadow, concealer, blush, highlighter, a skincare base, a skin care primer, a makeup base, or a makeup primer.
33. A cosmetic composition comprising:
   a. about 3% by weight of a polyglycerol surfactant,
   b. about 3.6% by weight of a higher alcohol,
   c. about 0.2% by weight of a fatty acid,
   d. about 0.02% by weight of a neutralization agent, and less than 15% by weight of a UV absorber.
34. The cosmetic composition of item 33, wherein the cosmetic composition further comprises about 0.5% by weight of a taurate based thickener and/or 0.2% of xanthan gum.
35. The cosmetic composition of item 33 or 34, wherein the cosmetic composition is essentially free of any cyclic silicone, linear silicone, silicone resin, or silicone elastomer.
36. The cosmetic composition of any one of items 33 to 35, wherein the polyglycerol surfactant, the higher alcohol, and the fatty acid are emulsifiers, wherein the emulsifiers are essentially free of PEG.
37. A method of treatment and/or care of the skin, hair, nails and/or mucous membranes of a subject comprising applying the cosmetic composition of any one of items 1 to 36 to the skin, hair, nails and/or mucous membranes of the subject.

## Claims

1. A cosmetic composition comprising:
a. a polyglycerol surfactant,
b. a higher alcohol,
c. a fatty acid, and
d. a neutralization agent,
wherein the cosmetic composition is an oil-in-water (O/W) emulsion, wherein the emulsion is substantially free of particles that are greater than 15 microns in size.

2. A cosmetic composition comprising:
a. about 0.5-5% by weight of a polyglycerol surfactant,
b. about 1-5% by weight of a higher alcohol,
c. about 0.05-0.5% by weight of a fatty acid, and
d. about 0.0005-0.5% by weight of a neutralization agent,
wherein the cosmetic composition is an oil-in-water (O/W) emulsion, wherein the emulsion is substantially free of particles that are greater than 15 microns in size.

3. The cosmetic composition of claim 1 or 2, comprising:
a. about 1-3% by weight of a polyglycerol surfactant,
b. about 2-3.8% by weight of a higher alcohol,
c. about 0.1-0.3% by weight of a fatty acid, and
d. about 0.05-0.2% by weight of a neutralization agent.

4. The cosmetic composition of any one of claims 1-3, wherein the cosmetic composition further comprises an UV absorber, preferably comprises less than 25% by weight of a UV absorber.

5. The cosmetic composition of any one of claims 1-4, wherein the cosmetic composition does not comprise a thickener.

6. The cosmetic composition of any one of claims 1-4, wherein the cosmetic composition further comprises at least one taurate based thickener and/or xanthan gum, preferably comprises about 0.05-2% by weight of a taurate based thickener and/or 0.05%-0.3% by weight of xanthan gum, wherein preferably the taurate based thickener is a copolymer of ammoniumacryloyldimethyltaurate and vinylpyrrolidone monomers (ammonium acryloyldimethyltaurate/vp copolymer).

7. The cosmetic composition of any one of claims 1-6, wherein the emulsion is substantially free of particles that are greater than 10 microns in size;
wherein the fatty acid is behenic acid;
wherein the neutralization agent is potassium hydroxide or sodium hydroxide;
wherein the higher alcohol is stearyl alcohol, behenyl alcohol or a combination thereof; and/or
wherein the polyglycerol surfactant is polyglycerol-10 stearate or polyglycerol-10 di-stearate.

8. The cosmetic composition of any one of claims 4-7, wherein the UV absorber is homosalate, octocrylene, ethylhexyl salicylate, butylmethoxy dibenzoylmethane, bis-ethylhexyloxyphenol methoxyphenyl triazine or a combination thereof and/or wherein the UV absorber absorbs ultraviolet light between about 290-400 nanometers.

9. The cosmetic composition of any one of claims 1-8, wherein the cosmetic composition further comprises a preservative, wherein the preservative is preferably phenoxyethanol.

10. The cosmetic composition of any one of claims 1-9, wherein the percent by weight ratio of the higher alcohol to the polyglycerol surfactant is between 3:5 and 18:5;
wherein the percent by weight ratio of the neutralization agent to the fatty acid is between 1:40 and 1:1;
wherein the cosmetic composition is essentially free of PEG;
wherein the cosmetic composition is essentially free of any silicones;
wherein the cosmetic composition is essentially free of cyclic silicones, linear silicones, silicone resins, silicone elastomers, and silicone-based surfactants;
wherein the cosmetic composition is a thixotropic cosmetic composition;
wherein the cosmetic composition is opaque;
wherein the cosmetic composition is chemically stable for at least 36 months at room temperature; and/or
wherein the cosmetic composition maintains a stabilized O/W emulsion.

11. The cosmetic composition of any one of claims 1-10, further containing one or more coloring materials and/or dermatological active ingredients, wherein the dermatological active ingredients preferably are emollients, humectants, hydrating active ingredients, antiaging ingredients, extracts or combinations thereof, and/or wherein the cosmetic product is preferably selected from a skin care base, suncare, sunscreen, lipstick, a foundation, lip gloss, eyeshadow, concealer, blush, highlighter, a skincare base, a skin care primer, a makeup base, or a makeup primer.

12. A cosmetic composition comprising:
a. about 3% by weight of a polyglycerol surfactant,
b. about 3.6% by weight of a higher alcohol,
c. about 0.2% by weight of a fatty acid,
d. about 0.02% by weight of a neutralization agent, and less than 15% by weight of a UV absorber.

13. The cosmetic composition of claim 12, wherein the cosmetic composition further comprises about 0.5% by weight of a taurate based thickener and/or 0.2% of xanthan gum.

14. The cosmetic composition of claim 12 or 13, wherein the cosmetic composition is essentially free of any cyclic silicone, linear silicone, silicone resin, or silicone elastomer; and/or wherein the polyglycerol surfactant, the higher alcohol, and the fatty acid are emulsifiers, wherein the emulsifiers are essentially free of PEG.

15. A method of treatment and/or care of the skin, hair, nails and/or mucous membranes of a subject comprising applying the cosmetic composition of any one of claims 1 to 14 to the skin, hair, nails and/or mucous membranes of the subject.
